# EUROPEAN PATENT SPECIFICATION

(11) **EP 1 957 084 B1**
(45) Date of publication and mention of the grant of the patent: **04.09.2013**
(21) Application number: 06824306.2
(22) Date of filing: 05.12.2006
(51) Int. Cl.: A61K 31/70, A23L 1/09, A23L 1/0524, A23L 1/0528, A23L 1/29

(54) **COMPOSITION CONTAINING OLIGOSACCHARIDES FOR THE TREATMENT / PREVENTION OF FEVER AND FEBRILE SEIZURES**
ZUSAMMENSETZUNG MIT OLIGOSACCHARIDEN ZUR BEHANDLUNG/PRÄVENTION VON FIEBER UND FIEBERANFÄLLEN
PRÉPARATION CONTENANT DES OLIGOSACCHARIDES POUR LE TRAITEMENT PROPHYLACTIQUE/THÉRAPEUTIQUE DE LA FIEBRE ET DES CRISES HYPERPYRÉTIQUES

(30) Priority: 06.12.2005 WO PCT/NL2005/050064
(43) Date of publication of application: 20.08.2008
(73) Proprietor: N.V. Nutricia, 2712 HM Zoetermeer (NL)
(72) Inventor: POTAPPEL - VAN 'T LAND, Belinda, NL-3775 KS Kootwijk (NL); GARSSEN, Johan, NL-3433 DA Nieuwegein (NL); BOEHM, Gunther, 61209 Echzell (DE); STAHL, Bernd, 61191 Rosbach-rodheim (DE)
(74) Representative: Nederlandsch Octrooibureau
(86) International application number: PCT/NL2006/050304
(87) International publication number: WO 2007/067053

(56) References cited:
- WO-A-00/08948
- WO-A-01/60378
- WO-A-2005/039597
- WO-A-2006/112714
- US-A1- 2003 022 863
- FANARO S ET ALACIDIC OLOGOSACCHARIDES FROM PECTIN HYDROLYSATE AS NEW COMPONENT FOR INFANT FORMULAE: EFFECT ON INTESTINAL FLORA, ST: "ool Characteristics, and pH" JOURNAL OF PEDIATRIC GASTROENTEROLOGY AND NUTRITION, vol. 41, August 2005 (2005-08), pages 186-190, XP009061086

## Description

### FIELD OF THE INVENTION

The present invention relates to the use of oligosaccharide mixtures for the treatment and/or prevention of infections.

### BACKGROUD OF THE INVENTION

Oligosaccharides, particularly galactooligosaccharides and fructopolysaccharides are often included in nutritional composition for their bifidogenic effects. Recently, new activities of specific oligosaccharides have been described.

WO 2005039597 relates to a method for enhancing the immune system and the treatment and/or prevention of immune system related disorders in a mammal, particularly newborns, said method comprising the administration of uronic acid oligosaccharide and neutral oligosaccharide. Food compositions suitable for use in the above method are also provided.

Boehm G et al (Prebiotics in infant formulas: immune modulators during infancy) Nutrafoods 2005; 4:51-57 describes that oral administration of GOS/FOS significantly stimulates the cellular (i.e., Th1/Th2) immune balance.

EP 1267891 provides a pharmaceutical or dietetic product, which serves for reducing and/or blocking the adhesion of pathogenic substances and organisms to eukaryotic cells, in particular mammalian cells. The product described contains at least one carbohydrate having an uronic acid unit on one of the ends thereof. Of the, terminal uronic acid units pertaining to the carbohydrates present, 10 to 100% are provided with a double bond that is especially situated between the C₄ and C₅ atom.

WO 2006/112714 discloses a method for the restoration of gastrointestinal flora, maintaining gastrointestinal health and preventing infections, by administration of a composition comprising uronic acid oligosaccharides, probiotic bacteria and optionally prebiotic oligosaccharides.

### SUMMARY OF THE INVENTION

It has now surprisingly been found that, besides the known action of oligosaccharides, galacturonic acid oligosaccharides neutral oligosaccharides can reduce the number of systemic viral genome copies in organs, particularly early after infection. Furthermore, it was found that the activity of natural killer cells was also increased after administration of the present oligosaccharides. This strongly suggests that the oligosaccharides stimulate the innate immunity. To date, only the stimulation of the adaptive immunity with oligosaccharides has been described.

The consequences of these findings are that the present oligosaccharides can be advantageously used to reduce the severity of symptoms such as fever.

The invention relates to the use of
i) galacturonic acid oligosaccharides prepared by enzymatic digestion of pectin with pectin lyase, pectic lyase, endopolygalacturonase and/or pectinase
ii) a galactose based neutral oligosaccharide and
iii) a fructose based neutral neutral oligosaccharide,
   for the manufacture of a nutritional composition for oral administration to infants, said composition comprising between 5 and 60 en% lipid, between 5 and 40 en% protein and between 15 and 90 en% carbohydrate, for the treatment and/or prevention of a symptom selected from the group consisting of fever and febrile seizures in an infant.

### DETAILED DESCRIPTION OF PREFFERED EMBODIMENTS

The present invention provides a method or the treatment and/or prevention of a symptom selected from the group consisting of fever, febrile seizures, said method comprising the administration a composition comprising galacturonic acid and neutral oligosaccharides.

### Uronic acid oligosaccharide

The term uronic acid saccharide as used in the present invention refers to an oligosaccharide wherein at least 50% of the residues are selected from the group consisting of guluronic acid, mannuronic acid, galacturonic acid and glucuronic acid. Preferably the uronic acid saccharide is an uronic acid oligosaccharide with a degree of polymerization (DP) of 2 to 100. The uronic acid saccharide comprises at least 50 % galacturonic acid based on total uronic acid residues in the uronic acid oligosaccharide, such uronic acid oligosaccharide is hereinafter referred to as "galacturonic acid oligosaccharide". More preferably, the present galacturonic acid oligosaccharide is hydrolysed pectin, preferably polygalacturonic acid, even more preferably prepared by hydrolysis of apple pectin, citrus pectin and/or sugar beet pectin.

In a preferred embodiment, the galacturonic acid oligosaccharides of the present invention comprises between 25 and 100 wt.% galacturonic acid oligosaccharides with a DP between 2 and 100 based on total weight of galacturonic acid, more preferably between 50 and 100 wt.%, even more preferably between 75 and 100 wt.%. Preferably the composition comprises between 25 and 100 wt.% galacturonic oligosaccharides with a DP between 2 and 50 based on total weight of galacturonic acid, more preferably between 50 and 100 wt.%, even more preferably between 75 and 100 wt.%.

The galacturonic acid oligosaccharides are prepared by enzymatic digestion of pectin with pectin lyase, pectic lyase, endopolygalacturonase and/or pectinase.

The galacturonic acid oligosaccharide may be methoxylated and/or amidated. The galacturonic acid oligosaccharide is preferably indigestible in the upper human intestinal tract and water-soluble.

In a preferred embodiment, at least one of the terminal hexose units of the galacturonic acid oligosaccharide has a double bond, which is preferably situated between the C₄ and C₅ position of the terminal hexose unit, i.e. between the carbon atoms in the ring to which R₄ and R₅ are attached. The double bond provides effectively protects against attachment of the pathogenic bacteria to the epithelium. Preferably one of the terminal hexose units comprises the double bond. The double bond at a terminal hexose unit is preferably obtained by enzymatically hydrolyzing pectin with lyase.

Preferably the galacturonic acid oligosaccharide has the structure I below, wherein the terminal hexose (left) preferably comprises a double bond. The hexose units other than the terminal hexose unit(s) are galacturonic acid units. The carboxylic acid groups on these units may be free or (partly) esterified, and preferably at least 10% is methylated (see below).

### Structure I: Polymeric galacturonic acid oligosaccharide

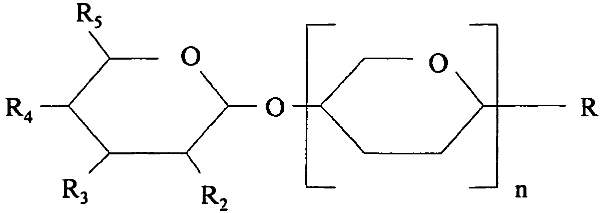

wherein:
R is preferably selected from the group consisting of hydrogen, hydroxy or acid group, preferably hydroxy; and
one selected from the group consisting of R₂, R₃, R₄ and R₅ represents free or esterified carboxylic acid and the remaining of R₂, R₃, R₄ and R₅ represent hydroxy and/or hydrogen; and
n is an integer and refers to the number of hexose units (see also Degree of Polymerisation (DP)). Suitably n is an integer between 1-249, preferably between 1 and 99, more preferably between 1 and 49.

In a further embodiment, a mixture of galacturonic acid oligosaccharides is used, which have a different DP and/or comprise both unsaturated and saturated terminal hexose units. Preferably at least 5%, more preferably at least 10%, even more preferably at least 25% of the terminal hexuronic units of the galacturonic acid oligosaccharide are unsaturated hexuronic units, as for example described above e.g. a terminal hexose unit of the uronic acid oligosaccharide with a double bond preferably situated between the C₄ and C₅ position. As each individual galacturonic acid oligosaccharide preferably comprises only one unsaturated terminal hexuronic unit, preferably less than 50% of the terminal hexuronic units is an unsaturated hexuronic unit (i.e. comprises a double bond).

A mixture of galacturonic acid oligosaccharides preferably comprises between 2 and 50% unsaturated terminal hexuronic units based on the total amount of terminal hexuronic units, preferably between 10 and 40%.

The galacturonic acid oligosaccharide can be derivatised. In one embodiment the galacturonic acid oligosaccharides are characterized by a degree of methylation above 20%, preferably above 50 % even more preferably above 70%. As used herein, "degree of methylation" (also referred to as DE or "degree of esterification") is intended to mean the extent to which free carboxylic acid groups comprised in the polygalacturonic acid chain have been esterified (e.g. by a methyl group). In another embodiment the galacturonic acid oligosaccharides have a degree of methylation above 20%, preferably above 50 % even more preferably above 70%.

### Concentration galacturonic acid oligosaccharides

The present composition is a nutritional composition, comprising fat, digestible carbohydrate and protein. The present nutritional composition preferably comprises between 0.01 and 5 grams galacturonic acid oligosaccharide with a DP of 2 to 250 per 100 gram dry weight of the nutritional composition, more preferably between 0.05 and 2 grams per 100 gram dry weight. The present nutritional composition preferably comprises between 0.01 and 5 grams galacturonic acid oligosaccharide with a DP of 2 to 250 (preferably DP of 2-100) per 100 gram dry weight of the nutritional composition, more preferably between 0.05 and 2 grams per 100 gram dry weight.

The present method preferably comprises the administration of between 0.05 and 10 grams galacturonic acid oligosaccharide with a DP of 2 to 100 per day, even more preferably between 0.1 and 5 grams galacturonic acid oligosaccharides per day.

### Neutral oligosaccharides

According to the present invention besides galacturonic acid saccharides, neutral oligosaccharides are used. Or in other words the composition for treatment and/or prevention a symptom selected from the group consisting of fever and febrile seizures further comprises neutral oligosaccharides. The term neutral oligosaccharides as used in the present invention refers to saccharides which have a degree of polymerization (DP) of saccharide units exceeding 2, more preferably exceeding 3, even more preferably exceeding 4, which are not or only partially digested in the intestine by the action of acids or digestive enzymes present in the human upper digestive tract (small intestine and stomach) but which are fermented by the human intestinal flora and preferably lack acidic groups. The neutral oligosaccharide is structurally (chemically) different from the uronic acid oligosaccharide.

The term neutral oligosaccharides as used in the present invention preferably refers to saccharides which have a degree of polymerisation preferably below 60 saccharide units, preferably below 40, even more preferably below 20, most preferably below 10.

The term saccharide units refers to units having a closed ring structure, preferably hexose, e.g. in pyranose or furanose form.

The neutral oligosaccharide preferably comprises at least 90%, more preferably at least 95% saccharide units selected from the group consisting of mannose, arabinose, fructose, fucose, rhamnose, galactose, β-D-galactopyranose, ribose, glucose, xylose and derivatives thereof, based on the total number of saccharide units comprised therein.

In one embodiment the present invention comprises the administration of a composition which comprises a neutral oligosaccharide selected from the group consisting of galactooligosaccharides, fructopolysaccharides and fructooligosaccharides; and a galacturonic acid oligosaccharide selected from the group consisting of short chain pectin and short chain alginate.

Short as in short chain pectin and short chain alginate refers to oligosaccharide derived from pectin or alginate with a DP between 2 and 10.

Suitable neutral oligosaccharides are preferably fermented by the gut flora. Suitable oligosaccharides and their production methods are further described in Laere K.J.M. (Laere, K.J.M., Degradation of structurally different non-digestible oligosaccharides by intestinal bacteria: glycosylhydrolases of Bi. adolescentis. PhD-thesis (2000), Wageningen Agricultural University, Wageningen, The Netherlands).

In the present galactooligosaccharides, preferably at least 50 % of the saccharide units are galactose. Transgalactooligosaccharides (TOS) are particularly suitable and are for example sold under the trademark Vivinal™ (Borculo Domo Ingredients, Netherlands).

The present method comprises the administration of 2 chemically distinct neutral oligosaccharides. The administration of galacturonic acid oligosaccharides combined with two chemically distinct neutral oligosaccharides provides an optimal effect.

Preferably the present method comprises the administration of an
- galacturonic acid oligosaccharides (see above);
- galactose based neutral oligosaccharide (>50 % of the saccharide units are galactose), preferably selected from the group consisting of galactooligosaccharide and transgalactooligosaccharide; and
- fructose based neutral oligosaccharides (>50% of the saccharide units are fructose), preferably inulin, fructan and/or fructooligosaccharide.

This composition is particularly suited for administration to infants in the age between 0-1 year.

The method comprises the administration of two chemically distinct neutral oligosaccharides, said chemically distinct oligosaccharides having a different DP and/or different average DP, preferably different average DP. In another embodiment administering chemically distinct neutral oligosaccharides with different average DP, provides an even more optimal immune-modulating effect. Preferably galactose based neutral oligosaccharide has an average DP between 2 and 10, and fructose based neutral oligosaccharides have an average DP between 10 and 60.

The neutral oligosaccharide is preferably administered in an amount of between 10 mg and 100 gram per day, preferably between 100 mg and 50 grams per day, even more preferably between 0.5 and 20 gram per day.

The acid- and neutral oligosaccharides act synergistically. Preferably the acid and neutral oligosaccharides are administered in a weight ratio of between 0.01:1 and 1:0.01, preferably in a weight ratio of between 0.1:1 and 1: 0.1.

### Foods

It was found that the galacturonic acid oligosaccharides neutral oligosaccharides can be advantageously applied in food, such as baby food, infant formula and clinical nutrition. Such food preferably comprises lipid, protein and carbohydrate and is preferably administered in liquid form. The term "liquid food" as used in the present invention includes dry food (e.g. powders) which are accompanied with instructions as to admix said dry food mixture with a suitable liquid (e.g. water).

Hence, the present invention relates to a nutritional composition which comprises between 5 and 60 en% lipid, between 5 and 40 en% protein, between 15 and 90 en% carbohydrate and the present galacturonic acid oligosaccharides in combination with the neutral oligosaccharides. Preferably the present nutritional composition preferably comprises between 10 and 60 en% lipid, between 5 and 40 en% protein and between 25 and 75 en% carbohydrate (en% is short for energy percentage and represents the relative amount each constituent contributes to the total caloric value of the preparation).

Such food preferably is in liquid form and has a limited viscosity. It was found that the foods comprising the galacturonic acid oligosaccharides, combined with the neutral oligosaccharides, provides a liquid nutrition with sufficiently low viscosity so it can be applied as e.g. liquid baby foods and liquid clinical food which can be fed through a teat, tube or a straw, while retaining the low viscosity. In a preferred embodiment, the present composition is orally administered to infants and in one embodiment has a viscosity below 600 mPas, preferably below 250 mPas, more preferably below 50 mPas, most preferably below 25 mPas at a shear rate of 100 s⁻¹ at 20°C. Whenever the term viscosity used in the present document, this refers to the physical parameter which is determined according to the following method:
The viscosity may be determined using a Carri-Med CSL rheometer. The used geometry is of conical shape (6 cm 2 deg acrylic cone) and the gap between plate and geometry is set on 55 µm. A linear continuous ramp shear rate is used from 0 to 150 s⁻¹ in 20 seconds.

Stool irregularities (e.g. hard stools, insufficient stool volume, diarrhoea) is a major problem in many babies that suffer from an childhood infection and receive liquid foods. The stool problems may be reduced by administering the present oligosaccharides in a liquid that has an osmolality between 50 and 500 mOsm/kg, more preferably between 100 and 400 mOsm/kg. In view of the above, it is also important that the liquid food does not have an excessive caloric density, however still provides sufficient calories to feed the subject. Hence, the liquid food preferably has a caloric density between 0.1 and 2.5 kcal/ml, even more preferably a caloric density of between 0.5 and 1.5 kcal/ml.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

Example 1: Effect of uronic acid oligosaccharide and neutral oligosaccharide on cytomegalovirus infection

To evaluate possible systemic protective effects of immune modulation induced by nutritional intervention, a model for murine cytomegalovirus (MCMV) infection was used. In this model, the effect of a prebiotic oligosaccharide mixture was investigated.

C57BL/6J mice were supplemented orally with a mixture comprising galacto-oligosaccharides, fructo-oligosaccharides and uronic acid oligosaccharides (GOS/FOS/AOS) two weeks prior and during systemic infection with MCMV. Immunomodulatory effects were analyzed by, a.o., delayed-type hypersensitivity (DTH) measurement as an *in vivo* parameter for T-helper 1 type of immunity. In addition, in several organs viral load was measured using a quantitative polymerase chain reaction technique (Q-PCR).

Within mice receiving the prebiotic mixture, MCMV DNA copy numbers were significantly reduced in multiple organs especially early after infection. Furthermore, a MCMV-specific DTH response could be detected in both groups. Yet, the time needed to develop a MCMV-specific DTH response differed significantly between groups. In mice receiving a placebo diet, DTH reached significance at day 6 post infection, whereas in mice receiving GOS/FOS/AOS, the onset of DTH response was delayed and reached significance at day 14. It is suggested that the delay in onset of DTH immunity is due to a lower pathogenic / antigenic load in supplemented mice.

In conclusion, this study suggests that supplementation with a prebiotic oligosaccharide mixture influences early innate immunity and as such affects the systemic MCMV infection in C57BL/6J mice.

The outcome of this study is indicative for the advantageous use of uronic acid saccharides, preferably combined with neutral oligosaccharides for the treatment and/or prevention of childhood infection and/or for the treatment and/or prevention a symptom selected from the group consisting of febrile sickness, febrile event, fever and febrile seizures.

### Example 2: Infant Nutrition

A liquid infant nutrition, prepared by admixing 13.9 g powder with water to yield 100 ml final product, said liquid product comprising per 100 ml:

| | |
|---|---|
| Energy: | 66 kcal |
| Protein: | 8 en% |
| | 1.3 g (comprising 0.5 g casein; 0.7 g whey; 0.072 g L-arginine) |
| Digestible Carbohydrates: | 44 en% |
| | 7.4 g (comprising 7.3 g lactose) |
| Fat: | 48 en% |

3.5 g (comprising 0.41 g linoleic acid ; 0.08 g α-linolenic acid; 0.012 g arachidonic acid; 0.002 g eicosapentanoic acid; 0.006 g docosahexaenoic acid; 1.4 g oleic acid; )

| | |
|---|---|
| Fibre: | 0.8 g (comprising 0.05 g fructopolysaccharide (Raftiline HP™, Orafti, Tienen, Belgium); 0.55 g transgalactooligosaccharides (Vivinal-GOS™ (Borculo Domo Ingredients, Netherlands); 0.20 g pectin hydrolysate prepared as described in EP 1373543, example 1. |
| Osmolarity: | 300 mOsmol/l |

The composition further comprises choline (6 mg/100 ml) and taurine (6.3 mg/100 ml); minerals and trace elements (including 2 mg zinc/100 ml) and vitamins in amounts in compliance with the international guidelines for infant milk formula.

## Claims

1. Use of
i) galacturonic acid oligosaccharides prepared by enzymatic digestion of pectin with pectin lyase, pectic lyase, endopolygalacturonase and/or pectinase
ii) a galactose based neutral oligosaccharide and
iii) a fructose based neutral oligosaccharide,
for the manufacture of a nutritional composition for oral administration to infants, said composition comprising between 5 and 60 en% lipid, between 5 and 40 en% protein and between 15 and 90 en% carbohydrate, for the treatment and/or prevention of a symptom selected from the group consisting of fever and febrile seizures in an infant.

2. Use according to claim 1, wherein the galacturonic acid oligosaccharide has a degree of polymerization of 2 to 100.

3. Use according to claim 1 or 2, wherein
a) the galactose based neutral oligosaccharide is selected from the group consisting of galactooligosaccharide and transgalactooligosaccharide and
b) the fructose based and/or glucose based neutral oligosaccharide is selected from the group consisting of inulin, fructopolysaccharide and fructooligosaccharide.

4. Use according to any one of the preceding claims, wherein the infants are in the age between 0-1 year.

## Patentansprüche

1. Verwendung von
i) Galacturonsäureoligosacchariden hergestellt durch enzymatische Verdauung von Pektin mit Pektinlyase, pektischer Lyase, Endopolygalacturonase und/oder Pektinase,
ii) einem auf Galaktose basierenden neutralen Oligosaccharid und
iii) einem auf Fructose basierenden neutralen Oligosaccharid
für die Herstellung einer Nahrungsmittelzusammensetzung zur oralen Einnahme durch Kinder, wobei die Zusammensetzung zwischen 5 und 60 En% Fett, zwischen 5 und 40 En% Protein und zwischen 15 und 90 En% Kohlenhydrate enthält, zur Behandlung und/oder Prävention eines Symptoms ausgewählt aus der Gruppe bestehend aus Fieber und Fieberkrämpfen bei einem Kleinkind.

2. Verwendung nach Anspruch 1, wobei das Galacturonsäureoligosaccharid einen Polymerisationsgrad von 2 bis 100 aufweist.

3. Verwendung nach Anspruch 1 oder 2, wobei
a) das auf Galaktose basierende neutrale Oligosaccharid ausgewählt ist aus der Gruppe bestehend aus Galactooligosaccharid und Transgalactooligosaccharid und
b) das auf Fructose basierende und/oder auf Glucose basierende neutrale Oligosaccharid ausgewählt ist aus der Gruppe bestehend aus Inulin, Fructopolysaccharid und Fructooligosaccharid.

4. Verwendung nach einem der vorangegangenen Ansprüche, wobei die Kleinkinder im Alter von 0-1 Jahr sind.

## Revendications

1. Utilisation
i) d'oligosaccharides d'acide galacturonique préparés par digestion enzymatique de pectine avec une pectine lyase, une lyase pectique, une endopolygalacturonase et/ou une pectinase
ii) d'un oligosaccharide neutre à base de galactose et
iii) d'un oligosaccharide neutre à base de fructose,
pour la fabrication d'une composition nutritionnelle destinée à une administration orale chez les nourrissons, ladite composition comprenant entre 5 et 60 % d'énergie en lipides, entre 5 et 40 % d'énergie en protéines et entre 15 et 90 % d'énergie en glucides, pour le traitement et/ou la prévention d'un symptôme sélectionné dans le groupe constitué par la fièvre et les convulsions fébriles chez le nourrisson.

2. Utilisation selon la revendication 1, dans laquelle l'oligosaccharide d'acide galacturonique a un degré de polymérisation de 2 à 100.

3. Utilisation selon la revendication 1 ou 2, dans laquelle
a) l'oligosaccharide neutre à base de galactose est sélectionné dans le groupe constitué par le galacto-oligosaccharide et le transgalacto-oligosaccharide et
b) l'oligosaccharide neutre à base de fructose et/ou à base de glucose est sélectionné dans le groupe constitué par l'inuline, le fructopolysaccharide et le fructo-oligosaccharide.

4. Utilisation selon l'une quelconque des revendications précédentes, dans laquelle les nourrissons ont un âge compris entre 0 et 1 an.
